# FASCICULE DE BREVET EUROPEEN

(11) **EP 2 487 235 B1**
(45) Date de publication et mention de la délivrance du brevet: **02.07.2014**
(21) Numéro de dépôt: 12167653.0
(22) Date de dépôt: 14.09.2005
(51) Int. Cl.: C12N 1/20, G01N 33/50, C12Q 1/14, C12Q 1/04, C12Q 1/42, C12Q 1/44

(54) **Milieu de detection de Streptococcus agalatiae en utilisant l'activité esterase**
Kulturmedium für den nachweise von Streptococcus agalatiae mittel Esteraseaktivität
Medium for detecting Streptococcus agalatiae using esterase activity

(30) Priorité: 16.09.2004 FR 0452068
(43) Date de publication de la demande: 15.08.2012
(62) Demande divisionnaire de: 05799851.0
(73) Titulaire: bioMérieux, 69280 Marcy-L'Etoile (FR)
(72) Inventeur: Robichon, Denis, 01150 Blyes (FR); Barbaux, Laurence, 01500 Ambérieu-en-Bugey (FR)

(56) Documents cités:
- EP-A- 0 881 284
- EP-A- 1 293 575
- CA-C- 1 322 733
- FR-A1- 2 708 286
- US-A- 4 259 442

## Description

La présente invention concerne le domaine de la détection et l'identification de *Streptococcus agalactiae.* Plus particulièrement, l'invention concerne l'utilisation de substrats d'estérase, éventuellement en association avec au moins un substrat d'α-glucosidase, de phosphatase, de β-cellobiosidase ou de N-acétyl-glucosaminidase, pour la détection et l'identification de *Streptococcus agalactiae.*

Le genre *Streptococcus* comporte de nombreuses espèces très répandues dans la nature, sur la peau et les muqueuses de l'homme et des animaux et sont à l'origine de multiples infections. Ce sont des bactéries ubiquistes que l'on retrouve à l'état libre dans le milieu extérieur (sol, air, eau), à l'état saprophyte ou à l'état de commensal chez l'homme et les animaux. Leurs localisations sont le rhinopharynx pour les streptocoques des groupes A, C, G, H et *salivarius,* l'intestin pour les streptocoques fécaux du groupe D et la cavité vaginale pour les streptocoques du groupe B. Leur rôle pathogène est extrêmement varié et dépend des espèces en cause et de leur localisation dans l'organisme.

Les streptocoques sont des coques Gram+, de 0,5 à 1 μm de diamètre, présentant un groupement en chaînette et immobiles. Catalase négative, à métabolisme fermentant, ils sont anaérobies facultatifs et sont sensibles aux variations de température (croissance optimale 37°C) et aux variations de pH (pH optimal 7).

*Streptococcus agalactiae* (ou streptocoque B) est reconnu comme l'un des principaux agents infectieux responsables de la mammite chez les bovidés. Chez l'homme, c'est essentiellement un saprophyte des voies génitales de la femme (vagin), mais il se retrouve également dans le rhinopharynx et dans l'intestin, notamment le rectum. Chez les adultes, la colonisation demeure souvent asymptomatique, mais *Streptococcus agalactiae* peut être responsable de septicémies, de pneumonies, de méningites, d'arthrites, d'infections urinaires et de suppurations profondes. Chez la femme enceinte ou après l'accouchement, l'infection peut conduire à des endométrites et à une stérilité.

Chez le nouveau-né, la contamination se produit *in utero* ou, le plus souvent, lors de l'accouchement par inhalation du liquide amniotique ou de secrétions vaginales. Une infection précoce apparaît souvent dès la naissance ou dans les premières heures de la vie. L'infection précoce est favorisée par la prématurité, la rupture des membranes et une forte colonisation du vagin de la mère. Le taux de mortalité dans ce type d'infection est très élevé (> 50%). Les infections tardives se traduisent généralement par des méningites (méningite du nourrisson) et des arthrites.

Le dépistage systématique du portage de *Streptococcus agalactiae* est recommandé en fin de grossesse, idéalement entre 34 et 38 semaines d'aménorrhée (35-37 semaines de grossesse), en raison notamment de sa prévalence (10% en France, soit au moins 75 000 femmes enceintes/an) et de ses conséquences lors des accouchements à terme, ce qui en fait un problème de santé publique.

Des milieux sélectifs et/ou des milieux permettant une orientation du diagnostic sont disponibles dans le commerce. Toutefois, ces milieux ont pour inconvénient qu'ils ne se suffisent pas à eux seuls pour le diagnostic de *Streptococcus agalactiae* et qu'il est nécessaire d'effectuer des tests complémentaires, tels que la mise en évidence de l'antigène du groupe B de Lancefield (polysaccharide avec présence dominante de rhamnose) et l'hydrolyse de l'hippurate (bouillon à l'hippurate).

Les milieux sélectifs les plus couramment utilisés sont le bouillon de Todd-Hewitt, bouillon d'enrichissement destiné à la recherche des streptocoques du groupe B chez la femme enceinte. Ce bouillon contient différents antibiotiques inhibant la plupart des germes Gram négatif de la flore d'accompagnement, tels que l'acide nalixidique et la gentamycine, ou l'acide nalixidique, la polymyxine et le cristal violet.

Après l'étape d'enrichissement, le bouillon de Todd-Hewitt complémenté en antibiotiques doit être repiqué sur des milieux destinés à la recherche des streptocoques (voir recommandations du CDC (Center for Disease Control), MMWR (Morbidity and Mortality Weekly Report), 16 août 2002, Vol. 51, n° RR-11).

Le milieu de Lim est un variant du bouillon Todd-Hewitt et il contient 1% d'extrait de levure, de l'acide nalixidique et de la colistine.

Une gélose Columbia contenant 5% de sang est également utilisée et permet notamment la mise en évidence du caractère β-hémolytique de *Streptococcus agalactiae.* Toutefois, ce caractère n'apparaît pas toujours : le halo d'hémolyse autour des colonies peut être étroit, donnant plutôt l'aspect α-, voire γ-hémolytique. En revanche, ce caractère devient net si au voisinage des colonies de *Streptococcus agalactiae,* se trouvent des colonies de *Staphylococcus aureus* (Camp-Factor).

Ces milieux sélectifs ont pour inconvénients qu'ils doivent être complétés par des tests biochimiques et/ou immunologiques.

Actuellement, le seul milieu sélectif prêt à l'emploi disponible dans le commerce, permettant l'isolement et l'identification directe de *Streptococcus agalactiae* à partir de prélèvements recto-vaginaux est le milieu Granada (Biolys SA). Ce milieu a pour caractéristique qu'il favorise la production d'un pigment caroténoïde par les souches de *Streptococcus agalactiae* du fait de la présence dans le milieu d'amidon soluble, protéose peptone n°3, glucose, pyruvate de sodium, sulfate de magnésium, méthotrexate, colistine, cristal violet, agar, sérum de cheval, Na₂HPO₄ anhydre, métronidazole, MOPS (acide morpholinopropanesulfonique) hémisodique, eau distillée et incubation en anaréobiose. Ce milieu a donc pour inconvénient que la détection directe de *Streptococcus agalactiae* se fait en condition anaérobie, ce qui n'est pas aisé à mettre en oeuvre. Par ailleurs, aucun milieu de détection contenant un ou plusieurs substrats enzymatiques n'est disponible.

Document EP 1 293 575 décrit un système de tests universels pour l'identification de multiples familles de microorganismes. Ainsi, ce document fait un catalogue mêlant en deux longues listes les microorganismes détectables et les substrats enzymatiques utilisables. Le document EP 1 293 575 suggère a l'homme du métier la combinaison de substrat suivante: peptidase + glycosidase + uréase + tryptophanase+ estérase + décarboxylase. De plus, dans ce document, on retrouve une grande liste de microorganismes a détecter.

Le document EP0881 284 décrit un milieu de culture pour la détection spécifique des entérobactéries. Le document EP0881 284, montre la détection des Salmonella et d'autres entérobactéries en utilisant le milieu BKD1, contenant un substrat d'estérase, et que cette détection leur est spécifique puisque ce milieu ne permet pas de détecter d'autres souches, telles que Streptococcus spp.

Le document FR2708286 décrit un milieu de culture comprenant au moins deux chromogènes. Des exemples de tels chromogènes sont décrits dans le document FR2708286. Ce document décrit un nombre important de substrats potentiels. Mais, le document FR2708286 ne décrit ni n'illustre la détection de *Streptococcus agalactiae.* FR2708286 illustre seulement la détection des Streptococcus D.

La Demanderesse a maintenant mis en évidence contre toute attente qu'il était possible d'utiliser des substrats enzymatiques, en particulier des substrats enzymatiques d'estérase, pour la détection spécifique et l'identification de *Streptococcus agalactiae.*

En effet, de façon surprenante, la Demanderesse a mis en évidence que seules les *Streptococcus agalactiae* parmi les espèces de bactéries les plus proches et les plus fréquemment rencontrées de manière associée n'étaient pas capables d'utiliser les substrats enzymatiques d'estérase de façon précoce (à moins de 18h après l'inoculation), de sorte qu'elles sont les seules à ne pas être révélées de façon précoce par les substrats d'estérase, par exemple en n'obtenant pas de modification des colonies dans le milieu de façon précoce, par exemple en n'obtenant pas de modification de la coloration des colonies dans le milieu lorsqu'on utilise un substrat d'estérase chromogène, sans que la coloration ne diffuse dans le milieu réactionnel, donc concentrée au niveau des colonies, sans toutefois que ces molécules n'aient aucun effet néfaste sur leur croissance.

En conséquence, ce substrat enzymatique a comme avantage supplémentaire que la lecture des résultats peut se faire de façon précoce, notamment à environ 18-20 h d'incubation, avec un très bon contraste.

Les substrats enzymatiques d'estérase appropriés aux fins de l'invention sont tout substrat connu de l'homme du métier permettant de mettre en évidence une telle activité enzymatique. De tels substrats peuvent être par exemple chromogéniques ou fluorescents et sont décrits par exemple dans le catalogue BIOSYNTH, Substrates and Reagents ou www.biosynth.com ou dans le catalogue GLYCOSYNTH, enzyme substrates catalogue ou www.glycosynth.co.uk.

A titre d'exemple de substrats d'estérase, on peut citer les dérivés d'indoxyl-octanoate, indoxyl-nonanoate ou indoxyl-décanoate, de préférence des dérivés indoxyl-octanoate, de préférence encore leurs dérivés halogénés, de préférence encore les dérivés chlorés et bromés tels que le 5-bromo-6-chloro-3-indoxyl-octanoate et le 5-bromo-4-chloro-3-indoxyl-octanoate pour lesquels la lecture est particulièrement précoce.

Comme une légère activité estérase est observée après 24h d'incubation (activité inférieure à 0,6 sur une échelle de 0 à 4), la détection des *Streptococcus agalactiae* peut être améliorée en ajoutant au moins un autre substrat enzymatique. Du fait de la propriété particulière des *Streptococcus agalactiae* à ne pas utiliser ou à utiliser très peu le substrat d'estérase, il est indifférent que l'autre substrat enzymatique soit ou non utilisé par *Streptococcus agalactiae* et les autres espèces. De plus, comme l'utilisation du substrat d'estérase par *Streptococcus agalactiae* n'est que très faible de sorte que cela ne modifie que faiblement l'aspect des colonies obtenues, nous indiquerons uniquement dans la suite que *Streptococcus agalactiae* ne sont pas capables d'utiliser le substrat d'estérase.

Ainsi, la présente invention a pour objet un milieu réactionnel comprenant ou constitué d'un substrat d'estérase que Streptococcus agalactiae n'est pas capable d'utiliser à moins de 18h après l'inoculation et (d')au moins un substrat enzymatique utilisé par *Streptococcus agalactiae,* différent d'un substrat d'estérase.

Les substrats enzymatiques autres qu'un substrat d'estérase (substrat non estérase) appropriés aux fins de l'invention sont tout substrat dont l'utilisation par une souche confère à la colonie un aspect différent de l'aspect obtenu lors de l'utilisation du substrat d'estérase. Un tel aspect différent est par exemple une coloration différente. Par ailleurs, ce substrat non estérase est tel que, lorsqu'une souche utilise à la fois ce substrat non estérase et le substrat d'estérase (souche différente de *Streptococcus agalactiae*), l'aspect des colonies obtenues (par exemple leur coloration) est également différent de l'aspect des colonies de *Streptococcus agalactiae.* En effet, lorsqu'on combine dans un milieu réactionnel à la fois un substrat d'estérase et un substrat non estérase utilisable par les souches de *Streptococcus agalactiae,* les souches de *Streptococcus agalactiae* sont alors négatives pour l'estérase et positives pour le substrat non estérase (on peut les noter -/+, la première partie de l'équation correspondant au substrat d'estérase et la deuxième partie correspondant au substrat non estérase), tandis que les autres souches sont capables d'utiliser soit uniquement le substrat d'estérase (elles sont +/-), soit à la fois le substrat d'estérase et le substrat non estérase (elles sont +/+). De même, lorsqu'on combine dans un milieu réactionnel à la fois un substrat d'estérase et un substrat non estérase non utilisable par les souches de *Streptococcus agalactiae,* les souches de *Streptococcus agalactiae* sont alors négatives pour l'estérase et négatives pour le substrat non estérase (elles sont -/-), tandis que les autres souches sont capables d'utiliser soit uniquement le substrat d'estérase (elles sont +/-), soit à la fois le substrat d'estérase et le substrat non estérase (elles sont +/+). En résumé, les souches de *Streptococcus agalactiae* sont toujours -/+ ou -/- tandis que les autres espèces sont toujours +/- ou +/+.

Ainsi, par exemple, si on combine un substrat d'estérase chromogène entraînant une coloration bleue des colonies lorsque la colonie considérée utilise le substrat, et un autre substrat enzymatique chromogène entraînant une coloration rose des colonies lorsque la colonie considérée utilise le substrat, on peut obtenir quatre types de coloration : soit rose, soit incolore à légèrement bleu, soit bleu, soit violet (rose+bleu). La coloration rose et l'aspect incolore à légèrement bleu sont uniquement représentatifs des *Streptococcus agalactiae* comme suit : soit la souche est capable d'utiliser le substrat non estérase et la colonie devient rose (souche -/+), soit elle n'est pas capable d'utiliser le substrat non estérase et la colonie reste incolore ou devient légèrement bleue (souche -/-). Les colorations bleu et violet sont représentatives des autres espèces comme suit : soit la souche est capable uniquement d'utiliser le substrat d'estérase et elle devient bleue (souche +/-), soit la souche est capable à la fois d'utiliser le substrat d'estérase et l'autre substrat enzymatique et elle devient rose et bleue, soit violette (souche +/+).

De même, si on combine un substrat d'estérase absorbant la fluorescence, entraînant une extinction de fluorescence lorsque la colonie considérée utilise le substrat, et un autre substrat enzymatique fluorescent entraînant une fluorescence au niveau des colonies lorsque la colonie considérée utilise le substrat, ce dernier substrat étant utilisé par *Streptococcus agalactiae,* on peut obtenir deux types de colonies : soit des colonies fluorescentes, soit des colonies faiblement à non fluorescentes. Les colonies fluorescentes sont uniquement représentatives des *Streptococcus agalactiae* car cette espèce est uniquement capable d'utiliser le substrat enzymatique autre que le substrat d'estérase. Les colonies faiblement à non fluorescentes sont représentatives des autres espèces comme suit : soit la souche est capable uniquement d'utiliser le substrat d'estérase et elle est non fluorescente, soit la souche est capable à la fois d'utiliser le substrat d'estérase et l'autre substrat enzymatique et elle est faiblement à non fluorescente.

Selon un mode de réalisation, de tels substrats autre qu'un substrat d'estérase appropriés aux fins de l'invention comprennent les substrats d'α-glucosidase, les substrats de phosphatase, les substrats de β-cellobiosidase, les substrats de N-acétyl-glucosaminidase et les substrats de β-glucosidase.

Les substrats enzymatiques d'α-glucosidase appropriés aux fins de l'invention sont tout substrat connu de l'homme du métier permettant de mettre en évidence une telle activité enzymatique. De tels substrats peuvent être par exemple chromogéniques ou fluorescents et sont décrits par exemple dans le catalogue BIOSYNTH, Substrates and Reagents ou www.biosynth.com ou dans le catalogue GLYCOSYNTH, enzyme substrates catalogue ou www.glycosynth.co.uk.

A titre d'exemple de substrat d'α-glucosidase, on peut citer les substrats à base de dérivés d'indoxyle, les substrats à base de dérivés d'umbelliférone et les substrats à base de dérivés de naphtol.

De préférence, le substrat enzymatique d'α-glucosidase approprié aux fins de l'invention est un substrat à base de dérivés d'indoxyle.

Des exemples de tels dérivés d'indoxyle comprennent les dérivés de 3-indolyl-α-D-glucopyranoside, de préférence des dérivés halogénés de ces composés. A titre d'exemples de dérivés de 3-indolyl-α-D-glucopyranoside halogénés, on peut citer le 6-bromo-3-indolyl-α-D-glucopyranoside, le 5-bromo-6-chloro-3-indolyl-α-D-glucopyranoside, le 5-bromo-4-chloro-3-indolyl-α-D-glucopyranoside, le 5-bromo-4-chloro-3-indolyl-N-méthyl-α-D-glucopyranoside et le 6-chloro-3-indolyl-α-D-glucopyranoside, ce dernier composé étant particulièrement préféré.

Les substrats enzymatiques de phosphatase appropriés aux fins de l'invention sont tout substrat connu de l'homme du métier permettant de mettre en évidence une telle activité enzymatique. De tels substrats peuvent être par exemple chromogéniques ou fluorescents et sont décrits par exemple dans le catalogue BIOSYNTH, Substrates and Reagents ou www.biosynth.com.

A titre d'exemple de substrat de phosphatase, on peut citer les substrats à base de dérivés d'indolyle, les substrats à base de dérivés d'umbelliférone et les substrats à base de nitrophényle.

De préférence, le substrat enzymatique de phosphatase approprié aux fins de l'invention est un substrat à base de dérivés d'indoxyle.

Des exemples de tels dérivés d'indoxyle comprennent les dérivés de 3-indolyle-phosphate tels que 5-bromo-4-chloro-3-indolyl phosphate, le 5-bromo-6-chloro-3-indolyl phosphate et le 6-chloro-3-indolyl-phosphate, ce dernier composé étant particulièrement préféré.

Les substrats enzymatiques de β-cellobiosidase appropriés aux fins de l'invention sont tout substrat connu de l'homme du métier permettant de mettre en évidence une telle activité enzymatique. De tels substrats peuvent être par exemple chromogéniques ou fluorescents et sont décrits par exemple dans le catalogue BIOSYNTH, Substrates and Reagents ou www.biosynth.com ou dans le catalogue GLYCOSYNTH, enzyme substrates catalogue ou www.glycosynth.co.uk.

A titre d'exemple de substrat de β-cellobiosidase, on peut citer les substrats à base de dérivés d'indolyle, les substrats à base de dérivés d'umbelliférone et les substrats à base de nitrophényle.

De préférence, le substrat enzymatique de β-cellobiosidase approprié aux fins de l'invention est un substrat à base de dérivés d'indoxyle.

Des exemples de tels dérivés d'indoxyle comprennent les dérivés de 3-indolyle-β-D-cellobioside tels que 6-chloro-3-indolyl-β-D-cellobioside et le 5-bromo-4-chloro-3-indolyl-β-D-cellobioside, ce dernier composé étant particulièrement préféré.

Les substrats enzymatiques de N-acétyl-glucosaminidase appropriés aux fins de l'invention sont tout substrat connu de l'homme du métier permettant de mettre en évidence une telle activité enzymatique. De tels substrats peuvent être par exemple chromogéniques ou fluorescents et sont décrits par exemple dans le catalogue BIOSYNTH, Substrates and Reagents ou www.biosynth.com ou dans le catalogue GLYCOSYNTH, enzyme substrates catalogue ou www.glycosynth.co.uk.

A titre d'exemple de substrat de N-acétyl-glucosaminidase, on peut citer les substrats à base de dérivés d'indoxyle, les substrats à base de dérivés d'umbelliférone et les substrats à base de nitrophényle.

De préférence, le substrat enzymatique de N-acétyl-glucosaminidase approprié aux fins de l'invention est un substrat à base de dérivés d'indoxyle.

Des exemples de tels dérivés d'indoxyle comprennent les dérivés de 3-indolyle-β-N-acétyl-glucosaminide tels que 5-bromo-6-chloro-3-indolyl-N-acétyl-β-D-glucosaminide, le 6-chloro-3-indolyl-N-acétyl-β-D-glucosaminide et le 5-bromo-4-chloro-3-indolyl-β-N-acétyl-glucosaminide, ce dernier composé étant particulièrement préféré.

Les substrats enzymatiques de β-glucosidase appropriés aux fins de l'invention sont tout substrat connu de l'homme du métier permettant de mettre en évidence une telle activité enzymatique. De tels substrats peuvent être par exemple chromogéniques ou fluorescents et sont décrits par exemple dans le catalogue BIOSYNTH, Substrates and Reagents ou www.biosynth.com ou dans le catalogue GLYCOSYNTH, enzyme substrates catalogue ou www.glycosynth.co.uk.

A titre d'exemple de substrat de β-glucosidase, on peut citer les substrats à base de dérivés d'indolyle, les substrats à base de dérivés d'umbelliférone et les substrats à base de nitrophényle.

De préférence, le substrat enzymatique de β-glucosidase approprié aux fins de l'invention est un substrat à base de dérivés d'indoxyle.

Des exemples de tels dérivés d'indoxyle comprennent les dérivés de 3-indolyle-β-D-glucopyranoside tels que 5-bromo-4-chloro-3-indolyl-β-D-glucopyranoside, 5-bromo-6-chloro-3-indolyl-β-D-glucopyranoside, 6-chloro-3-indolyl-β-D-gluco-pyranoside et le 5-bromo-4-chloro-3-indolyl-N-méthyl-β-D-glucopyranoside.

Selon un mode de réalisation, le milieu réactionnel comprend i) un substrat d'estérase que Streptococcus agalactiae n'est pas capable d'utiliser à moins de 18h après l'inoculation et ii) un substrat de phosphatase ou un substrat d'α-glucosidase, l'association substrat d'estérase/substrat de phosphatase étant préférée.

Selon un autre mode de réalisation, le milieu réactionnel comprend, outre le substrat d'estérase et le substrat de phosphatase ou d'α-glucosidase, un substrat enzymatique choisi parmi un substrat de β-cellobiosidase, un substrat de N-acétyl-glucosaminidase et un substrat de β-glucosidase, de préférence un substrat de β -cellobiosidase et un substrat de N-acétyl-glucosaminidase.

Le milieu réactionnel tel qu'utilisé dans le procédé de l'invention est donc un milieu réactionnel de détection du fait de la présence d'au moins un substrat enzymatique.

Ce milieu réactionnel peut soit servir uniquement de milieu de révélation, soit de milieu de culture et de révélation. Dans le premier cas, la culture des microorganismes est effectuée avant ensemencement et, dans le deuxième cas, le milieu réactionnel constitue également le milieu de culture.

Le milieu réactionnel peut être solide, semi-solide ou liquide. Par milieu solide ou semi-solide, on entend par exemple un milieu gélifié.

L'agar est le milieu traditionnel solide en microbiologie pour la culture des microorganismes, mais il est possible d'utiliser de la gélatine ou de l'agarose. Un certain nombre de préparation sont disponibles dans le commerce, comme par exemple l'agar Columbia, la gélose Trypcase-soja, la gélose Mac Conkey, la gélose Sabouraud ou plus généralement celles décrites dans le Handbook of Microbiological Media (CRC Press).

La quantité d'agar dans le milieu réactionnel est de 2 à 40g/l. Pour les milieux solides, la quantité d'agar est de préférence de 9 à 25g/l, de préférence encore de 12 à 14g/l. Pour les milieux semi-solides, la quantité d'agar est de préférence de 2 à 6g/l.

Les substrats enzymatiques de l'invention sont utilisables dans une large gamme de pH, notamment entre pH 5,5 et 10.

La concentration de ou des substrat(s) enzymatique(s) dans le milieu réactionnel est comprise entre 10 et 2000 mg/l, de préférence entre 50 et 500 mg/l, de préférence encore entre 80 et 400 mg/l, ce qui constitue un mode de réalisation préféré de l'invention.

Bien entendu, l'homme du métier déterminera la concentration du ou des substrat(s) enzymatique(s) dans le milieu dans cette gamme en fonction du substrat choisi. Ainsi, dans la mesure où le substrat d'estérase utilisé est le 5-bromo-4-chloro-3-indolyl-octanoate, on préfère une concentration comprise entre 100 et 400 mg/l.

Le milieu réactionnel utile aux fins de l'invention peut également comprendre d'autres composants utiles pour améliorer la spécificité et/ou la sensibilité du procédé de l'invention.

Ainsi, selon un mode de réalisation de l'invention, le milieu réactionnel comprend des solutions phosphate telles que des solutions de Na₂HPO₄ et K₂HPO₄.

En effet, l'utilisation de telles solutions phosphate permet d'améliorer de façon sensible la lisibilité du milieu qui se traduit soit par un renforcement de la netteté de coloration, soit par une augmentation de l'expression et/ou de la détection de l'activité phosphatase à 18h.

La concentration de telles solutions phosphate est comprise entre 0,3 et 1,5 g/l pour chaque solution, une concentration de 0,5 g/l étant préférée.

Le milieu réactionnel peut également contenir un mélange d'inhibiteurs pour inhiber ou limiter la croissance des souches indésirables, telles que les souches Faux positif, par exemple *Candida* ou *Staphylococcus saprophyticus,* sans modifier la sensibilité de détection du milieu.

A ce titre, le mélange réactionnel peut contenir un mélange d'antibiotiques. L'ajout d'antibiotiques dans le milieu réactionnel permet entre autre un gain de temps car l'identification de *Streptococcus agalactiae* se fait directement.

Des exemples d'antibiotiques qui conviennent aux fins de l'invention comprennent l'aztréonam et l'amphotéricine B. Ces antibiotiques sont disponibles dans le commerce auprès de ICN, Squibb ou Sigma.

La quantité de chaque antibiotique dans le milieu réactionnel varie en fonction de l'antibiotique concerné et sera facilement déterminée par l'homme du métier.

Le milieu réactionnel peut également comprendre un ou plusieurs éléments en combinaison, tels que des acides aminés, des peptones, des hydrates de carbone, des nucléotides, des minéraux, des vitamines, des tensioactifs, des tampons, des sels de phosphate, d'ammonium, de sodium, de métaux. Des exemples de milieux sont décrits dans les demandes de brevet de la Demanderesse, EP 656 421 et WO99/09 207.

La mise en oeuvre du procédé de la description peut être effectuée selon les étapes suivantes consistant à :
a) inoculer un milieu réactionnel tel que défini précédemment, avec tout ou partie de l'échantillon,
b) incuber le milieu inoculé,
c) révéler la présence d'au moins une activité estérase seule ou en combinaison avec au moins une autre activité enzymatique différente d'une activité estérase, ce qui constitue un autre objet de l'invention.

Les étapes d'inoculation et d'incubation sont largement connues de l'homme du métier.

Par exemple, la température d'incubation peut être de 37°C. S'agissant de l'atmosphère d'incubation, elle est préférentiellement aérobie.

La révélation est mise en oeuvre à l'oeil nu par visualisation d'un changement de coloration ne diffusant pas dans le milieu réactionnel, donc concentrée au niveau des colonies. Dans le cas de la révélation de la fluorescence, on utilise les dispositifs de lecture de la fluorescence connus de l'homme du métier.

Les échantillons biologiques à analyser sont tout échantillon clinique susceptible de contenir des *Streptococcus agalactiae,* comme un prélèvement vaginal, un prélèvement d'urine ou tout autre échantillon dont l'analyse peut aider un clinicien à poser un diagnostic.

L'invention sera mieux comprise à l'aide des exemples suivants donnés à titre illustratif et non limitatif.

### Exemple 1: Détection de Streptococcus agalactiae à l'aide de substrats enzymatiques d'estérase

### 1.1 Préparation des milieux réactionnels

On a préparé les milieux réactionnels en mélangeant de l'extrait coeur-cervelle (4,84 g/l ; Solabia), de l'infusion de viande (1,96 g/l ; Solabia), de la biothione (1 g/l ; Solabia), de la biotrypcase (7,2 g/l ; Solabia), du carbonate de sodium (0,3 g/l ; VWR), du pyruvate de sodium (2 g/l ; Fluka), du tampon HEPES (0,4 g/l ; Sigma), de la peptone de lactalbumine (2 g/l ; DMV), du glucose (1 g/l ; Merck), de l'agar américain (2 g/l ; Sobigel) et de l'agar européen (12 g/l ; Roko).

Après autoclavage 15 min à 121°C, on a ajouté un substrat enzymatique d'estérase tel qu'indiqué ci-dessous à raison de 0,3 g/l ; puis on a refroidi au bain-marie à 50°C :
- 5-bromo-4-chloro-3-indolyl-octanoate (X-C8 ; Inalco), qui donne une coloration turquoise lorsqu'il est utilisé, et
- 5-bromo-6-chloro-3-indolyl-octanoate (Magenta-C8 ; Inalco), qui donne une coloration rose-rouge quand il est utilisé.

On a ensuite coulé les milieux en boite de Petri pour l'inoculation ultérieure avec des souches de bactéries.

### 1.2 Ensemencement des souches de microorganismes

Trois souches de *Streptococcus agalactiae* et trois souches d'autres bactéries, toutes issues de la collection de la Demanderesse, mises en suspension dans de l'eau physiologique, ont été ensemencées pour donner des colonies isolées sur chacun des milieux. Les boîtes ont été incubées à 37 °C pendant 48 heures. Les colonies formées ont été examinées visuellement après 18, 24 et plus de 40 heures d'incubation. La coloration de ces colonies, la croissance, ainsi que l'intensité de cette coloration (représentative de l'activité estérase) ont été notées.

### 1.3 Résultats

Les résultats sont donnés dans le tableau 1 ci-après et sont exprimés :
- en croissance (C) avec indication de la taille en mm,
- en couleur (Co) avec T= Turquoise, R= Rose ou Rouge,
- en intensité (I) de coloration en se basant sur une échelle arbitraire allant de 0 à 4, 0 correspondant à une absence d'activité et 4 correspondant à la présence d'une coloration très intense,
- selon le temps d'incubation en heures (T).

**Tableau 1**

| **Souches (n° d'accès)** | | **X-C8** | | | **Magenta-C8** | | |
|---|---|---|---|---|---|---|---|
| | **T** | **C** | **Co** | **I** | **C** | **Co** | **I** |
| *Streptococcus agalactiae* (7611003) | 18 | 1,2 | | | 1,2 | | |
| | 24 | 2 | T | 0,3 | 2 | R | 0,3 |
| | > 40 | 2,5 | T | 2,3 | 2,5 | R | 1,7 |
| *Streptococcus agalactiae* (0101060) | 18 | 0,4 | | | 0,4 | | |
| | 24 | 0,7 | T | 0,3 | 0,7 | R | 0,3 |
| | > 40 | 1,3 | T | 3 | 1,3 | R | 2 |
| *Streptococcus agalactiae* (8904053) | 18 | | | | 0,3 | | |
| | 24 | 0,2 | | | 0,5 | | |
| | > 40 | 0,3 | T | 0,3 | 1 | | |
| *Enterococcus faecalis* (0008192) | 18 | 0,8 | T | 1,7 | 0,8 | R | 1 |
| | 24 | 2 | T | 3 | 1,8 | R | 1,7 |
| | > 40 | 2 | T | 3,5 | 2 | R | 3,5 |
| *Enterococcus faecium* (7611005) | 18 | 0,5 | T | 2 | 0,7 | R | 1 |
| | 24 | 1 | T | 3 | 1,7 | R | 2,7 |
| | > 40 | 1 | T | 3 | 1,7 | R | 3 |
| *Staphylococcus epidermidis* (7509009) | 18 | 0,5 | T | 2 | 0,5 | R | 2 |
| | 24 | 1,5 | T | 3 | 1 | R | 3 |
| | > 40 | 1,5 | T | 3 | 1,3 | R | 3,5 |

Les résultats mettent en évidence que les streptocoques B peuvent être détectés précocement en utilisant un substrat enzymatique d'estérase car ils présentent une activité nulle à très faible à 18-24h.

### Exemple 2 : Détection de Streptococcus agalactiae à l'aide d'un substrat d'estérase et d'un substrat d'α-glucosidase ou de phosphatase

On a répété le mode opératoire ci-dessus décrit dans l'exemple 1, à ceci près qu'on a ajouté, en même temps que 0,3 g/l du substrat d'estérase X-C8, 0,3 g/l de 6-chloro-3-indolyl-α-D-glucopyranoside (Rose-α-Glu), ou bien 0,3 g/l de 6-chloro-3-indolyl-phosphate (Rose-P), lesquels donnent une coloration rose lorsqu'ils sont utilisés.

Les résultats sont donnés dans le tableau 2 ci-dessous dans lequel on donne la croissance, la coloration et l'intensité, comme dans l'exemple 1, et où R=Rose/Rouge, RM=Rose-Marron, T=Turquoise, V=Vert, Vi=Violet, B= Bleu, GVi=Gris-Violet et GB= Gris-Bleu.

**Tableau 2**

| **Souches (n° d'accès)** | | **X-C8 + Rose-alpha-Glu** | | | **X-C8 + Rose-P** | | |
|---|---|---|---|---|---|---|---|
| | **T** | **C** | **Co** | **I** | **C** | **Co** | **I** |
| *Streptococcus agalactiae* (7611003) | 18 | 1,3 | R | 3 | 1 | R | 3 |
| | 24 | 1,3 | R | 3 | 1,7 | R | 4 |
| | > 40 | 2 | R | 4 | 2 | R | 4 |
| *Streptococcus agalactiae* (8709013) | 18 | 0,2 | R | 2 | 0,5 | R | 3 |
| | 24 | 0,3 | R | 2 | 0,5 | R | 3,5 |
| | > 40 | 1 | R | 4 | 1,7 | R | 4 |
| *Streptococcus agalactiae* (7702055) | 18 | 1 | R | 2 | 0,8 | R | 3 |
| | 24 | 1,7 | RM | 2,7 | 1,3 | R | 4 |
| | > 40 | 1,7 | R | 4 | 1,7 | R | 4 |
| *Enterococcus faecium* (7611005) | 18 | 1,5 | T | 3 | 1,7 | GB | 3 |
| | 24 | 1,7 | T | 3 | 1,7 | B | 3,5 |
| | > 40 | 1,8 | T | 4 | 2 | GVi | 4 |
| *Staphylococcus epidermidis* (7509009) | 18 | 0,7 | V | 3 | 0,6 | GVi | 3,5 |
| | 24 | 1,3 | GB | 3,5 | 1,3 | GVi | 3,5 |
| | > 40 | 1,3 | GB | 3,5 | 1,5 | GVi | 4 |
| *Staphylococcus aureus* (9202070) | 18 | 3 | GVi | 3 | 2 | Vi | 4 |
| | 24 | 3 | Vi | 4 | 3 | Vi | 4 |
| | > 40 | 3 | Vi | 4 | 3 | Vi | 4 |

Ce tableau met en évidence que la détection des souches de *Streptococcus agalactiae* est améliorée lorsqu'on utilise un substrat d'estérase chromogène en combinaison avec un autre substrat enzymatique chromogène, différent d'un substrat d'estérase, et utilisable par les souches de *Streptococcus agalactiae.*

### Exemple 3 : Détection de Streptococcus agalactiae à l'aide d'un substrat d'estérase, d'un substrat de phosphatase et d'un substrat de β-cellobiosidase

On a répété le mode opératoire décrit dans l'exemple 2, en utilisant 0,3 g/l de X-C8, 0,2 g/l de Rose-P, à ceci près qu'on a également ajouté 0,08 g/l de 5-bromo-4-chloro-3-indolyl-β-D-cellobioside (Cellobio) en même temps que les autres substrats, ainsi que 0,5 g/l de Na₂HPO₄ et 0,5 g/l de K₂HPO₄ avant autoclavage.

On a utilisé, à titre de milieu témoin, un milieu avec uniquement du X-C8 et du Rose-P.

Les résultats sont donnés dans le tableau 3 ci-dessous dans lequel on donne la croissance, la coloration et l'intensité, comme dans l'exemple 1, et où R=Rose/Rouge, Ma=Mauve, Vi=Violet, B=Bleu, GB=Gris-Bleu et Vl=Violine.

**Tableau 3**

| **Souches (n° d'accès)** | | **Témoin** | | | **X-C8 + Rose-P + Cellobio** | | |
|---|---|---|---|---|---|---|---|
| | **T** | **C** | **Co** | **I** | **C** | **Co** | **I** |
| *Streptococcus agalactiae* (0101060) | 18 | 0,7 | R | 1,7 | 0,7 | R | 1,3 |
| | 24 | 0,7 | R | 4 | 0,7 | R | 3 |
| | > 40 | 1,5 | R | 4 | 1,5 | R | 4 |
| *Streptococcus agalactiae* (7701031) | 18 | 1,3 | R | 4 | 1 | R | 4 |
| | 24 | 1,5 | R | 4 | 1,5 | R | 4 |
| | > 40 | 1,5 | R | 4 | 1,5 | R | 4 |
| *Streptococcus agalactiae* (7702055) | 18 | 1 | R | 2 | 1 | R | 2 |
| | 24 | 1,5 | R | 4 | 1,5 | R | 4 |
| | > 40 | 1,7 | R | 4 | 1,7 | R | 4 |
| *Streptococcus anginogus* (8507046) | 18 | 0,3 | | | 0,3 | B | 0,5 |
| | 24 | 0,5 | R | 0,1 | 0,4 | B | 1,3 |
| | > 40 | 1 | R | 2,3 | 1 | B | 2,7 |
| *Enteroccocus faecium* (0002043) | 18 | 1,5 | Ma | 1,7 | 1,3 | B | 3 |
| | 24 | 1,7 | Ma | 3 | 1,5 | GB | 4 |
| | > 40 | 2 | Vi | 4 | 2 | V1 | 4 |

Les résultats obtenus dans le tableau 3 mettent en évidence une amélioration de la spécificité de détection de *Streptococcus agalactiae* par rapport aux autres souches lorsqu'on utilise trois substrats enzymatiques dont un substrat d'estérase.

### Exemple 4 : Détection de Streptococcus agalactiae à l'aide d'un substrat d'estérase, d'un substrat de phosphatase et d'un substrat de N-acétyl-glucosaminidase

On a répété le mode opératoire décrit dans l'exemple 3 à ceci près qu'on a utilisé 0,4 g/l de 5-bromo-4-chloro-3-indolyl-β-N-acétyl-glucosaminide (X-NAGlu) à la place du Cellobio.

Le milieu témoin est identique au milieu testé, à ceci près qu'il ne contient pas de X-NAGlu.

Les résultats sont donnés dans le tableau 4 ci-dessous dans lequel on donne la croissance, la coloration et l'intensité, comme dans l'exemple 1, et où R=Rose/Rouge, B=Bleu, GR=Gris-Rose et Mg=Magenta.

**Tableau 4**

| **Souches (n° d'accès)** | | **Témoin** | | | **X-C8 + Rose-P + X-NAGlu** | | |
|---|---|---|---|---|---|---|---|
| | **T** | **C** | **Co** | **I** | **C** | **Co** | **I** |
| *Streptococcus agalactiae* (7611003) | 18 | 0,5 | R | 3 | 0,5 | R | 2,3 |
| | 24 | 1 | R | 4 | 1 | R | 3 |
| | > 40 | 1,2 | R | 4 | 1,2 | R | 4 |
| *Streptococcus agalactiae* (7701031) | 18 | 0,5 | R | 2,7 | 0,5 | R | 2,7 |
| | 24 | 0,7 | R | 4 | 0,7 | R | 4 |
| | > 40 | 0,7 | R | 4 | 0,7 | R | 4 |
| *Enterobacter clocae* (0010003) | 18 | 1,7 | R | 2,3 | 1,7 | GR | 2 |
| | 24 | 2 | R | 3 | 2 | B | 3 |
| | > 40 | 2,5 | B | 4 | 3 | B | 4 |
| *Enterococcus faecium* (0002043) | 18 | 0,5 | GR | 2 | 0,5 | B | 3 |
| | 24 | 0,8 | GR | 2,7 | 0,8 | B | 3,5 |
| | > 40 | 1 | Mg | 4 | 1 | B | 4 |

Les résultats dans ce tableau mettent en évidence une amélioration de la spécificité de détection de *Streptococcus agalactiae* par rapport aux autres souches lorsqu'on utilise trois substrats enzymatiques dont un substrat d'estérase.

### Exemple 5 : Détection de Streptococcus agalactiae à l'aide d'un substrat d'estérase, d'un substrat de phosphatase et d'un substrat de β-glucosidase

On a répété le mode opératoire décrit dans l'exemple 4 à ceci près qu'on a utilisé 0,08 g/l de 5-bromo-4-chloro-3-indolyl-β-D-glucopyranoside (X-β-Glu) et 0,3 g/l de 5-bromo-4-chloro-3-indolyl-N-méthyl-β-D-glucopyranoside (GreenA-β-Glu) à la place du X-NAGlu.

Le milieu témoin est identique au milieu testé, à ceci près qu'il ne contient pas de X-β-Glu ni de GreenA-β-Glu.

Les résultats sont donnés dans le tableau 5 ci-dessous dans lequel on donne la croissance, la coloration et l'intensité, comme dans l'exemple 1, et où R=Rose/Rouge, Ma=Mauve, Vi=Violet, B=Bleu et GB=Gris-Bleu.

**Tableau 5**

| **Souches (n° d'accès)** | | **Témoin** | | | **X-C8 + Rose-P + X-□-Glu** | | | **X-C8 + Rose-P + GreenA-□-Glu** | | |
|---|---|---|---|---|---|---|---|---|---|---|
| | **T** | **C** | **Co** | **I** | **C** | **Co** | **I** | **C** | **Co** | **I** |
| *Streptococcus agalactiae* (9001001) | 18 | 0,4 | | | 0,3 | | | 0,2 | | |
| | 24 | 0,5 | R | 0,5 | 0,5 | R | 0,3 | 0,4 | R | 0,3 |
| | > 40 | 1,7 | R | 4 | 1,3 | R | 4 | 1,5 | R | 4 |
| *Streptococcus agalactiae* (7701031) | 18 | 1,3 | R | 4 | 1,3 | R | 4 | 1 | R | 4 |
| | 24 | 1,5 | R | 4 | 1,5 | R | 4 | 1,5 | R | 4 |
| | > 40 | 1,5 | R | 4 | 1,5 | R | 4 | 1,5 | R | 4 |
| *Streptococcus agalactiae* (7702055) | 18 | 1 | R | 2 | 1 | R | 2 | 1 | R | 2 |
| | 24 | 1,5 | R | 4 | 1,5 | R | 4 | 1,3 | R | 4 |
| | > 40 | 1,7 | R | 4 | 1,7 | R | 4 | 1,5 | R | 4 |
| *Streptococcus anginogus* (8507046) | 18 | 0,3 | | | 0,3 | B | 3 | 0,3 | B | 0,5 |
| | 24 | 0,5 | R | 0,1 | 0,4 | B | 4 | 0,4 | B | 2 |
| | > 40 | 1 | R | 2,3 | 1 | B | 4 | 1 | B | 3,5 |
| *Enteroccocus faecium* (0002043) | 18 | 1,5 | Ma | 1,7 | 1,5 | B | 4 | 1,5 | GB | 4 |
| | 24 | 1,7 | Ma | 3 | 1,5 | B | 4 | 1,7 | GB | 4 |
| | > 40 | 2 | Vi | 4 | 2 | B | 4 | 2 | GB | 4 |

Les résultats obtenus dans le tableau 5 mettent en évidence une amélioration de la spécificité de détection de *Streptococcus agalactiae* par rapport aux autres souches lorsqu'on utilise trois substrats enzymatiques dont un substrat d'estérase.

### Exemple 6: Amélioration de la sensibilité de détection par ajout de solution phosphate

On a répété le mode opératoire décrit dans l'exemple 1, à ceci près qu'on a ajouté, en même temps que 0,3 g/l du substrat d'estérase X-C8, 0,3 g/l de Rose-P, ainsi que 0,5 g/l de Na₂HPO₄ et 0,5 g/l de K₂HPO₄.

A titre de milieu témoin, on a utilisé le même milieu, mais sans solution phosphate.

Les résultats sont donnés dans le tableau 6 ci-dessous dans lequel on donne la croissance, la coloration et l'intensité, comme dans l'exemple 1, et où R=Rose et Mg=Magenta.

**Tableau 6**

| **Souches (n° d'accès)** | | **Témoin** | | | **Milieu avec solution phosphate** | | |
|---|---|---|---|---|---|---|---|
| | **T** | **C** | **Co** | **I** | **C** | **Co** | **I** |
| *Streptococcus agalactiae* (7611003) | 18 | 1,3 | R | 3 | 1,3 | Mg | 3,5 |
| | 24 | 1,7 | Mg | 4 | 1,7 | Mg | 4 |
| | > 40 | 1,8 | Mg | 4 | 1,8 | Mg | 4 |
| *Streptococcus agalactiae* (0101060) | 18 | 0,4 | R | 0,5 | 0,5 | Mg | 3 |
| | 24 | 0,5 | Mg | 4 | 0,7 | Mg | 3,5 |
| | > 40 | 1,5 | Mg | 4 | 1,5 | Mg | 4 |
| *Streptococcus agalactiae* (7702055) | 18 | 1 | Mg | 3 | 1 | R | 3,5 |
| | 24 | 1,5 | Mg | 4 | 1,5 | Mg | 4 |
| | > 40 | 1,5 | Mg | 4 | 1,5 | Mg | 4 |

Les résultats dans ce tableau 6 mettent en évidence soit une amélioration de la netteté de coloration dès 18h, soit une augmentation de l'expression des souches de *S*. *agalactiae.*

### Exemple 7: Comparaison de la sensibilité et la spécificité de détection de S. agalactiae en utilisant un milieu contenant un substrat d'estérase selon l'invention et les milieux disponibles dans le commerce

Pour cette étude de sensibilité et de spécificité, on a utilisé un milieu selon l'invention préparé comme décrit dans l'exemple 1, contenant 0,3 g/l de X-C8, ainsi que : 0,2 g/l de Rose-P, 0,08 g/l de Cellobio, 0,5 g/l de Na₂HPO₄, 0,5 g/l de K₂HPO₄, 0,012 g/l d'Aztréonam et 0,004 g/l d'Amphotéricine B.

Comme milieu de comparaison, on a utilisé le milieu Granada (ref 10 077, BIOLYS, France) (Milieu Granada).

On a ensemencé 69 souches de microorganismes, dont 14 de *Streptococcus agalactiae,* on a laissé incuber à 37°C jusqu'à 24h et à température ambiante au-delà. On a visualisé les colonies comme décrit précédemment. La confirmation des colonies suspectes caractéristiques de Streptocoque B, c'est-à-dire apparaissant roses/rouges, a été réalisée par test d'agglutination en utilisant le réactif Slidex Strepto Kit selon les recommandations du fournisseurs (bioMérieux, France). Les colonies non caractéristiques, c'est-à-dire autres que roses ou ayant la coloration caractéristique mais donnant une réponse négative au test d'agglutination (souches faux positif), ont été identifiées par les Galeries ID 32 Strep (bioMérieux, France).

Les résultats sont exprimés en % de bon diagnostic par rapport à tous les tests en termes de sensibilité et spécificité et sont donnés dans le tableau 7 ci-après, le % de sensibilité correspondant au nombre de vrais positifs détectés sur le milieu par le nombre total de vrais positifs à détecter (*100) et le % de spécificité correspondant au nombre de vrais négatifs détectés sur le milieu par le nombre total de vrais négatifs à détecter (* 100).

**Tableau 7**

| | **Sensibilité et spécificité de détection de *S. agalactiae* en %** | | | | | |
|---|---|---|---|---|---|---|
| | **Milieu Granada** | | | **Milieu de l'invention** | | |
| | **18h** | **24h** | **> 40h** | **18h** | **24h** | **> 40h** |
| **Sensibilité sans enrichissement** | 50 | 50 | 50 | 79 | 79 | 93 |
| **Sensibilité avec enrichissement** | 50 | 50 | 50 | 79 | 86 | 93 |
| **Spécificité sans enrichissement** | 100 | 100 | 100 | 87 | 82 | 80 |
| **Spécificité avec enrichissement** | 100 | 100 | 100 | 89 | 93 | 82 |

Les résultats indiqués dans ce tableau mettent en évidence l'amélioration de la sensibilité de détection des Streptocoques B (*Streptococcus agalactiae*) en utilisant le procédé de l'invention. Par ailleurs, ils montrent également que le milieu de détection de l'invention possède également une bonne spécificité, spécificité améliorée après enrichissement du fait d'un passage en bouillon de Todd-Hewitt 18-24 heures à 35-37°C avec ou sans 5% de CO₂ avant ensemencement de la gélose (voir recommandations du CDC (Center for Disease Control), MMWR (Morbidity and Mortality Weekly Report), 16 août 2002, Vol. 51, n° RR-11).

### Exemple 8 : Utilisation du milieu à partir d'échantillons cliniques

Pour cette étude, on a utilisé le milieu selon l'invention tel que préparé comme décrit précédemment dans l'exemple 7.

Un total de 134 échantillons/écouvillons provenant de prélèvements vaginaux ou endocervicaux de femmes enceintes a été utilisé dans cette étude.

Chaque écouvillon a été émulsifié dans 1 ml d'eau physiologique stérile et 100 µl de cette solution ont été déposés d'une part sur une Gélose Columbia contenant 5% de sang de cheval et d'autre part sur le milieu utilisé dans le procédé de l'invention. Par ailleurs, 100 µl de la solution précédente ont été utilisés pour inoculer un bouillon Todd Hewitt. Après 20 heures d'incubation à 37°C et en aérobiose, la gélose au sang et le milieu de l'invention ont été ensemencés à partir du bouillon Todd Hewitt puis incubés 20 h à 37°C en aérobiose.

La confirmation des colonies suspectes caractéristiques de Streptocoque B, c'est-à-dire apparaissant roses/rouges, a été réalisée par test d'agglutination en utilisant le réactif Slidex Strepto Kit selon les recommandations du fournisseurs (bioMérieux, France).

Parmi les 134 échantillons, 112 ont été ensemencés sur les milieux gélosés d'une part directement à partir de la suspension en eau physiologique et d'autre part après enrichissement en bouillon Todd Hewitt. Les 22 échantillons restant ont été ensemencés sur les milieux gélosés uniquement directement à partir de la suspension en eau physiologique.

Les résultats, exprimés en pourcentage moyen de sensibilité et spécificité sont présentés dans le tableau 8 ci-après.

**Tableau 8**

| | Gélose Columbia | Gélose invention |
|---|---|---|
| Sensibilité | 95 | 100 |
| Spécificité | 90 | 99,5 |

Les résultats du tableau 8 ci-dessus montrent que le milieu de l'invention, utilisé avec des échantillons cliniques, permet une amélioration de la sensibilité et de la spécificité de détection des *Streptococcus agalactiae.* En effet, 20/20 prélèvements contenant du *Streptococcus agalactiae* sont détectés sur le milieu de l'invention contre 19 sur le milieu Columbia et il n'y a qu'un seul résultat faux + sur le milieu estérase contre 24 sur la gélose Columbia. On peut même noter que les résultats sont meilleurs que lorsqu'on a testé le milieu avec les souches de laboratoire.

## Revendications

1. Milieu réactionnel comprenant (i) un substrat enzymatique d'estérase que *Streptococcus agalactiae* n'est pas capable d'utiliser à moins de 18h après inoculation, (ii) un substrat enzymatique choisi parmi les substrats de β-cellobiosidase, les substrats de N-acétyl-glucosaminidase et les substrats de β-glucosidase, et (iii) un substrat de phosphatase.

2. Milieu réactionnel selon la revendication 1, **caractérisé en ce que** le substrat (ii) est un substrat de β-cellobiosidase.

3. Milieu réactionnel selon la revendication 1, **caractérisé en ce que** le substrat (ii) est un substrat de N-acétyl-glucosaminidase.

4. Milieu réactionnel selon la revendication 1, **caractérisé en ce que** le substrat (ii) est un substrat de β-glucosidase.

5. Milieu réactionnel selon la revendication 4, **caractérisé en ce que** le substrat β-glucosidase est un substrat d'indoxyle.

6. Milieu réactionnel selon l'une quelconque des revendications 1 à 5, **caractérisé en ce que** le substrat d'estérase est choisi parmi les dérivés d'indoxyle octanoate, de indoxyle nonanoate et de indoxyle décanoate.

7. Milieu réactionnel selon l'une quelconque des revendications 1 à 6, **caractérisé en ce que** la concentration en chaque substrat enzymatique est comprise entre 10 et 2000 mg/l.

8. Milieu réactionnel selon l'une quelconque des revendications L à 7, **caractérisé en ce qu'**il comprend également des solutions phosphate.

9. Milieu réactionnel selon la revendication 8, **caractérisé en ce que** les solutions phosphate sont choisies parmi Na₂HPO₄ et K₂HPO₄.

10. Milieu réactionnel selon l'une quelconque des revendications 1 à 9, **caractérisé en ce qu'**il comprend également un mélange d'inhibiteurs pour inhiber ou limiter la croissance des souches indésirables.

11. Milieu réactionnel selon la revendication 10, **caractérisé en ce que** les inhibiteurs sont des antibiotiques.

12. Milieu réactionnel selon la revendication 11, **caractérisé en ce que** les antibiotiques sont aztréonam et amphotericine B.

## Patentansprüche

1. Ein Reaktionsmedium, beinhaltend (i) ein Esterase-Enzymsubstrat, das *Streptococcus agalactiae* bei weniger als 18 h nach der Inokulation nicht verwenden kann, (ii) ein Enzymsubstrat, das ausgewählt ist aus β-Cellobiosidasesubstraten, N-Acetyl-Glucosaminidasesubstraten und β-Glucosidasesubstraten, und (iii) ein Phosphatasesubstrat.

2. Reaktionsmedium gemäß Anspruch 1, **dadurch gekennzeichnet, dass** das Substrat (ii) ein β-Cellobiosidasesubstrat ist.

3. Reaktionsmedium gemäß Anspruch 1, **dadurch gekennzeichnet, dass** das Substrat (ii) ein N-Acetyl-Glucosaminidasesubstrat ist.

4. Reaktionsmedium gemäß Anspruch 1, **dadurch gekennzeichnet, dass** das Substrat (ii) ein β-Glucosidasesubstrat ist.

5. Reaktionsmedium gemäß Anspruch 4, **dadurch gekennzeichnet, dass** das β-Glucosidasesubstrat ein Indoxylsubstrat ist.

6. Reaktionsmedium gemäß einem der Ansprüche 1 bis 5, **dadurch gekennzeichnet, dass** das Esterasesubstrat ausgewählt ist aus Indoxyloctanoat-, Indoxylnonanoat- und Indoxyldecanoatderivaten.

7. Reaktionsmedium gemäß einem der Ansprüche 1 bis 6, **dadurch gekennzeichnet, dass** die Konzentration in jedem Enzymsubstrat zwischen 10 und 2000 mg/! beträgt.

8. Reaktionsmedium gemäß einem der Ansprüche 1 bis 7, **dadurch gekennzeichnet, dass** es ebenfalls Phosphatlösungen beinhaltet.

9. Reaktionsmedium gemäß Anspruch 8, **dadurch gekennzeichnet, dass** die Phosphatlösungen aus Na₂HPO₄ und K₂HPO₄ ausgewählt sind.

10. Reaktionsmedium gemäß einem der Ansprüche 1 bis 9, **dadurch gekennzeichnet, dass** es ebenfalls ein Gemisch aus Hemmstoffen zur Hemmung oder Beschränkung des Wachstums unerwünschter Stämme beinhaltet.

11. Reaktionsmedium gemäß Anspruch 10, **dadurch gekennzeichnet, dass** die Hemmstoffe Antibiotika sind.

12. Reaktionsmedium gemäß Anspruch 11, **dadurch gekennzeichnet, dass** die Antibiotika Aztreonam und Amphotericin B sind.

## Claims

1. A reaction medium comprising (i) an esterase enzyme substrate which *Streptococcus agalactiae* is not capable of using at less than 18 hours after inoculation, (ii) an enzyme substrate selected from β-cellobiosidase substrates, N-acetyl-glucosaminidase substrates and β-glucosidase substrates, and (iii) a phosphatase substrate.

2. The reaction medium according to Claim 1, **characterised in that** the substrate (ii) is a β-cellobiosidase substrate.

3. The reaction medium according to Claim 1, **characterised in that** the substrate (ii) is an N-acetyl-glucosaminidase substrate.

4. The reaction medium according to Claim 1, **characterised in that** the substrate (ii) is a β-glucosidase substrate.

5. The reaction medium according to Claim 4, **characterised in that** the β-glucosidase substrate is an indoxyl substrate.

6. The reaction medium according to any one of claims 1 to 5, **characterised in that** the esterase substrate is selected from derivatives of indoxyl octanoate, indoxyl nonanoate and indoxyl decanoate.

7. The reaction medium according to any one of claims 1 to 6, **characterised in that** the concentration of each enzyme substrate is between 10 and 2000 mg/l.

8. The reaction medium according to any one of claims 1 to 7, **characterised in that** it also comprises phosphate solutions.

9. The reaction medium according to Claim 8, **characterised in that** the phosphate solutions are selected from Na₂HPO₄ and K₂HPO₄.

10. The reaction medium according to any one of claims 1 to 9, **characterised in that** it also comprises a mixture of inhibitors for inhibiting or limiting the growth of the undesirable strains.

11. The reaction medium according to Claim 10, **characterised in that** the inhibitors are antibiotics.

12. The reaction medium according to Claim 11, **characterised in that** the antibiotics are aztreonam and amphotericin B.
